# EUROPEAN PATENT APPLICATION

(11) **EP 4 411 748 A1**
(43) Date of publication of application: **07.08.2024**
(21) Application number: 23154710.0
(22) Date of filing: 02.02.2023
(51) Int. Cl.: G16H 30/20, G16H 40/63, A61B 5/055, A61B 6/03, A61B 6/00, G01R 33/54

(54) **CONTROL OF A MEDICAL IMAGING SYSTEM USING AN IMAGING CHAIN COMPONENT**

(71) Applicant: Koninklijke Philips N.V., 5656 AG Eindhoven (NL)
(72) Inventor: LIPS, Oliver, Eindhoven (NL); JOHNSON, Mark Thomas, Eindhoven (NL); VOGTMEIER, Gereon, 5656AG Eindhoven (NL); WEISS, Steffen, Eindhoven (NL); LEUSSLER, Christoph Günther, Eindhoven (NL); KOEHLER, Thomas, Eindhoven (NL)
(74) Representative: Philips Intellectual Property & Standards

(57) **Abstract**

Disclosed here is a medical apparatus (100, 200, 400, 500, 600) comprising an imaging chain component (102, 102', 102") configured to be integrated into an imaging chain of a medical imaging system (205). The imaging chain is configured for acquiring measurement data (218) during operation of the medical imaging system, wherein the imaging chain component comprises: a local memory (108) storing local machine executable instructions, a local computational system (106), and a local data communication interface (110) configured for forming a local data connection (202) for exchanging data between the local computational system and the medical imaging system. The execution of the local machine executable instructions causes the computational system to control the medical imaging system to initiate acquisition (306) of the measurement data via the local data connection.

## Description

### FIELD OF THE INVENTION

The invention relates to medical imaging, in particular to the upgrade of medical imaging systems.

### BACKGROUND OF THE INVENTION

Various medical imaging techniques such as Magnetic Resonance Imaging (MRI), Computed Tomography, Positron Emission Tomography, and Single Photon Emission Tomography enable detailed visualization of anatomical structure of a subject. A common feature of all of these imaging modalities is that components are complex and are often times difficult to upgrade or modify.

United States patent publication US 11,435,412 B2 discloses one or more peripheral components that are attached to a medical imaging system. One or more devices are attached to the one ore more peripheral components and configured to communicate data with a controller of the medical imaging system.

### SUMMARY OF THE INVENTION

The invention provides for a medical apparatus, a computer program, and a method in the independent claims. Embodiments are given in the dependent claims.

In one aspect the invention provides for a medical apparatus that comprises an imaging chain component that is configured to be integrated into an imaging chain of the medical imaging system. An imaging chain as used herein encompasses components which are used to physically measure measurement data for a medical imaging system. These could be components that are used to control and generate radio-frequency or ionizing radiation. They may also be components that are used to measure or acquire data. These for example could be an X-ray detector, an X-ray tube, or a high voltage X-ray generator of a of a computed tomography (CT) scanner. or diagnostic X-ray system. Other examples include a coil or other component of a magnetic resonance imaging system. The imaging chain is configured for acquiring measurement data during the operation of the medical imaging system.

The imaging chain component comprises a local memory storing machine-executable instructions. The imaging chain component further comprises a local computational system. The imaging chain component further comprises a local data communication interface for forming a local data connection for exchanging data between the local computational system and the medical imaging system. Execution of the local machine-executable instructions causes the computational system to control the medical imaging system to initiate acquisition of the measurement data via the local data connection. This embodiment may be beneficial because when the imaging chain component is upgraded this may result in an upgrade or improvement of the medical imaging system.

In various embodiments the imaging chain component may also provide additional data for configuring the medical imaging system. For example, the imaging chain component may provide configuration data which is used to configure the medical imaging system to operate or use the imaging chain component.

By having the imaging chain component initiate the acquisition of the measurement data, the imaging chain component functionally becomes a controller of the medical imaging system. This may be beneficial because it may remove the need to upgrade a controller when upgrading the imaging chain component.

The initiation of the acquisition of the measurement data via or using the local data connection may take different forms in different examples. In one example, the local computational system may supply configuration data or control commands to a controller of the medical imaging system, which is then later triggered by the initiation of the acquisition of the measurement data. In other examples, the imaging chain component could function as the controller for the medical imaging system. When the acquisition of the measurement data is initiated by the imaging chain component it is initiated by providing the control commands to components of the medical imaging system other than the imaging chain component. As such other parts of the medical imaging system whose operation must also be modified by introducing the imaging chain component can receive appropriate control commands.

The local data connection may be formed using different wired or wireless protocols. For example the local data connection may in some examples be formed using a fiber optic, wired network, or other connection. In other examples the local data connection may be formed using Blue Tooth, Wi-Fi, or other radio communication protocol.

In another embodiment the imaging chain component comprises a network interface configured for forming a network connection with a remote reconstructor system. In examples, the network interface that forms the network connection is separate from the local data connection. The imaging component then forms a local data connection for controlling the medical imaging system and then forms the network connection with the remote reconstructor system separately. Execution of the local machine-executable instructions further causes the local computational system to accumulate the measurement data in the local memory. Execution of the local machine-executable instructions further causes the local computational system to transmit the measurement data to the remote reconstructor system. This embodiment may be beneficial because in this case the other components of the medical imaging system have been bypassed for the reconstruction of medical images from the measurement data.

This may have the benefit of providing a means of updating or improving the reconstruction of the measurement data without making modifications to the medical imaging system. For example, it may enable an older medical imaging system to be upgraded to modern standards by simply replacing the imaging chain component.

In another embodiment the local memory further comprises system configuration data for configuring the medical imaging system to acquire the measurement data with the imaging chain. Execution of the machine-executable instructions further causes the computational system to configure the medical imaging system by sending the configuration data to the medical imaging system via the local data connection. This may for example occur before the medical imaging system initiates acquisition of the measurement data. This embodiment may be beneficial because it provides a means for the imaging chain component to automatically configure the medical imaging system. This may include components of the medical imaging system other than the imaging chain component also.

In another embodiment the imaging chain component further comprises a user interface connection. Execution of the local machine-executable instructions further causes the computational system to receive modification data via the user interface connection. Execution of the local machine-executable instructions further causes the computational system to modify the configuration data before sending the configuration data to the medical imaging system. In this embodiment the user interface connection enables a connection that enables the user of the medical imaging system to control and update specific acquisitions of measurement data using a user interface that is provided on the imaging chain component. This provides a means of providing an alternative or replacement control system for the medical imaging system. The user interface connection could for example be used to connect to another computational device or computer. This may include such things as a tablet, a workstation, a cell phone or other computational device. This may enable such things as for example an app being provided on a mobile computational device which may be used to control the function of the medical imaging system once the imaging chain component has been introduced into the medical imaging system. The user interface connection may enable additional functionality such as pushing updates or configuration changes to the imaging chain component. This may enable updating of the entire medical apparatus or changing the configuration of the entire medical apparatus by pushing updates or configuration data to the imaging chain component.

In another embodiment the local memory further comprises system control commands configured for controlling the operation of the medical imaging system during acquisition of the measurement data. Execution of the machine-executable instructions further causes the computational system to acquire the measurement data by controlling the medical imaging system via the local data connection with the system control commands. In this embodiment, instead of the medical imaging system being configured and then having its operation triggered by the imaging chain component, the imaging chain component instead functionally controls the other components of the medical imaging system. This embodiment may be beneficial because it may provide a means of completely updating the control of a medical imaging system with the mere replacement of an imaging chain component.

In another embodiment the imaging chain component is a data measurement component. A data measurement component as used herein may encompass a component of an imaging chain that measures directly or indirectly the measurement data. There may be many benefits in incorporating the control of the medical imaging system into a data measurement component. For example, if the imaging chain component is a sensor or comprises a sensor then the data is acquired and then may be processed directly in the imaging chain component. This may eliminate the need to move data in and out of the existing medical imaging system. This for example may save bandwidth and large amounts of time. This embodiment may also be of benefit because it may provide for updated networking capabilities to an existing or older medical imaging system. Another potential benefit is that the data measurement component may perform some kind of preprocessing such as averaging or filtering of the measurement data as it is acquired and then used to directly reconstruct a medical image or transmit it over the network connection to the remote reconstructor system. This may for example be useful in greatly accelerating the function of the medical imaging system.

In another embodiment the medical imaging system comprises a magnetic resonance imaging system. The measurement data comprises k-space data. This may be particularly beneficial when the data measurement component is used to measure or record k-space data. Magnetic resonance imaging systems may acquire large amounts of k-space data even for a simple image. The replacement of a data measurement component in a magnetic resonance imaging system may provide for a means of preprocessing the k-space data before it is reconstructed, a means for efficiently offloading the k-space data to a remote reconstructor system, or even reconstruction of the magnetic resonance image using the data measurement component itself.

In another embodiment the data measurement component is a magnetic resonance imaging coil.

In another embodiment the data measurement component is a wireless magnetic resonance imaging coil.

In another embodiment the data measurement component is a magnetic resonance imaging coil interface configured for receiving a magnetic resonance imaging coil.

These embodiments may be beneficial because magnetic resonance imaging coils are used to receive the radio-frequency signals from a subject during a magnetic resonance imaging examination. The digitization of these radio-frequency signals is the k-space data.

In another embodiment the magnetic resonance imaging coil interface is incorporated into a subject support that is configured for supporting at least a portion of the subject in an imaging zone of the magnetic resonance imaging system. This for example may be beneficial because the subject support may be changed and this may provide for a means of updating the magnetic resonance imaging system as well as possibly providing a means for reconstructing magnetic resonance images or using them to be reconstructed by a reconstructor.

In the above embodiments the medical apparatus comprises an MRI system where RF coil (detector) functions as the imaging chain component that controls the MRI scanner.

In a first example an RF detector coil is the imaging chain component and acts as master device, which controls an MRI scanner by providing input for scan planning and setup.

Optionally, the imaging chain component may also collect scan data (k-space data).

In this example the detector (coil or interface) may be connected to the cloud/a computer network for receiving and transmitting data and display on "simple viewing station" or other type of user interface. The system may operate including one or more of the following features:
- Universal RF coil (detector) connected to the network transmits and receives information from/to the cloud.
- Information from the coil is used to setup and plan the scan. In this context a user interface (UI) on the host computer may be opened to adapt and confirm the scan planning.
- The scanner becomes a "stupid" executor of the scanning process
- Data is stored and transferred to the cloud by the coil/device

Such an approach may have the following advantages:
- Potentially vendor agnostic: The scan controlling and data harvesting device can be (in principle) independent from the scanner
- Data flow (scan planning/ data collection/ reconstruction) can bypass the scanner completely
- Upgrade / downgrade of the system is done via the exchange of a key component
- The required parameter setting for best quality operation of the key component comes with the key component that controls the system accordingly and requests the necessary information as for example the system feedback, the calibration data and the system data
- The imaging chain component may be the component with the biggest innovation capability (e.g. the detector and detector data handling). With the exchange of the key component the upgradability of a system is defined and the innovation cycle could be faster in case no other system adaptation would be necessary (as other system components could remain unchanged e.g., the main field coil in MRI) Also, adaptation of data processing, data handling and organization of data flow which might change more often than mechanics updates could be organized by an update of the "data generating component" (i.e., the imaging chain component)

Some MR systems may have separate coil interfaces that may comprise processing and/or sampling capacity and may be known as smart coil interfaces. One or more of these coil interfaces may be the imaging chain component. These interfaces may become more complex including digitalisation, preprocessing of data and optionally scanner control as a master. The example includes a multichannel digital connector /interface box. Analogue coils may be connected to the interface and the interface contains digitalisation and processing. The number of connected coils and their configuration (size) control and configure the scanner. The interface can also be integrated in the patient bed/support so that the patient support is considered as a peripheral unit and acts as a master.

In another embodiment the data measurement component is an X-ray detector. This embodiment may be beneficial because the changing of an X-ray detector may require a change in the way in which the measurement data is used to reconstruct a finished medical image.

In another embodiment the X-ray detector is a computed tomography detector.

In another embodiment the X-ray detector is a digital X-ray detector.

In another embodiment the X-ray detector is a flat-panel X-ray detector. For example, a flat-panel X-ray detector may be inserted into an X-ray machine where a film cassette would have been. Incorporating a control system into such an X-ray detector may be a useful way of changing an old obsolete X-ray system into a modem digital computer controlled X-ray system.

In another embodiment the X-ray detector is a flat-panel wireless X-ray detector.

In another embodiment the imaging chain component is an X-ray source.

In another embodiment the imaging chain component is an X-ray tube.

In another embodiment the imaging chain component is an X-ray tube power supply.

In another embodiment the imaging chain component is a high-voltage generator.

In another embodiment the imaging chain component is a controllable collimator.

In another embodiment the medical imaging system further comprises an X-ray-based diagnostic imaging system.

In another example, the medical imaging system is a Digital X-ray (DXR) system. DXR system where x-ray detector controls DXR scanner

In some examples the imaging chain component is an x-ray detector, e.g., a wireless flat panel x-ray detector, is the peripheral device acting as master device, which controls a DXR system by providing input for scan planning and setup. Optionally the master device may also collect scan data.

Specifically, such a DXR system may comprise one or more of the following features:
- a mobile wireless DXR detector (e.g. in a mobile/bed side DXR system) which
   - controls the trigger for an x-ray tube/generator including parameter setting
   - controls the mechanical movement of table/system mechanics and collimator (depending on system type) and
   - sends data to either its "own" processing station (or alternatively an extra tablet as master including cloud processing if necessary).

This embodiment may have the benefit that the detector initially has the raw data (measurement data) (like an MR RF coil). OEM detectors may use this to offer alternative iterative or AIbased reconstructions in parallel to the standard reconstruction on the system.

In another embodiment, the X-ray detector is a detector for a computed tomography (CT) system controlled by the imaging chain component (X-ray detector). As an example, a CT detector module (X-ray detector) is the master controller in a platform-based CT system. Depending on the type of module (16 line, 32 line, 64 line, 128 line, 256 line and if it is a conventional detector and/or a spectral / photon counting detector) the detector unit as master component controls the settings of the slave components like the collimator, the parameters for the x-ray tube (focal spot control) and the generator fitting to the scan protocol settings. The master unit determines the allowed/useful parameter settings for the slave components depending on the hardware configuration.

The advantage of having the detector as a master controller (imaging chain component) with the latest control and scan software is interesting for CT systems where exchange of the detector allows for upgrade and/or reconfiguration while the other components like collimator, x-ray tube, and high voltage generator stays the same or can be adapted with modules like e.g., the data transmission infrastructure (slipring) with having more parallel channels for larger bandwidth.

Software updates may come together with the detector module as an exchangeable module and the remaining compatible components are then configured and calibrated according to the geometry and system setting of the detector with respect to size, acquisition rate, collimation, and operation.

The advantage is the flexibility in reconfiguration of a CT scanner with the exchange of the key imaging chain component only. The console remains the user interface and sends the patient and application specific information while the installed detector module provides with the best available scan options offered by the detector module including available scan speed (i.e., collimation) depending on detector size, image information (spectral/non-spectral) depending on detector technology and patient requirements.

Also the data organization with the defined request for reconstruction in the system / in the hospital cloud or the cloud could be initiated by the detector. As a consequence, the replacement of the detector module directly upgrades/downgrades the complete functionality of the CT scanner.

In another example in C-arm based 3D image generation or CT scanner that use a flat panel detector (x-ray detector) the detector could control the x-ray system including collimation but also could control the rotation of the gantry/C-arc and the movement of the table if required.

In another embodiment the imaging component further comprises a mechanical housing configured for being attached to the medical imaging system. The local memory is enclosed by the mechanical housing. The local data connection is at least partially enclosed by the mechanical housing. The local computational system is enclosed by the mechanical housing. This embodiment may be beneficial because it is a component which is attached to the medical imaging system. It provides a means of updating or improving the functionality of the medical imaging system by attaching an imaging chain component to it mechanically.

In another embodiment the medical apparatus comprises the medical imaging system.

In another embodiment the medical imaging system is a magnetic resonance imaging system.

In another embodiment the medical imaging system is a positron emission tomography system.

In another embodiment the medical imaging system is a single-photon emission tomography system.

In another embodiment the medical imaging system is a computed tomography system.

In another embodiment the medical imaging system is a digital fluoroscope system.

In another embodiment the medical imaging system is a digital X-ray system.

In another embodiment the medical imaging system is a tomographic medical imaging system.

In another embodiment the medical imaging system is a radiological medical imaging system.

In other embodiments the medical apparatus may comprise multiple imaging modalities. The updating of the imaging chain component for the medical imaging system may also be used to update and control the functionality of the other medical imaging modalities.

In another embodiment the medical imaging system is a combined magnetic resonance imaging system and positron emission tomography system.

In another embodiment the medical imaging system further comprises a combined medical magnetic resonance imaging system and a computed tomography system.

In embodiments where there is a hybrid scanner, as for example a PET-CT / PET-MRI system where one component of one system could also control the other system. In some examples a hierarchy definition, the master-slave definition may be negotiated.

In another embodiment the medical apparatus further comprises other components. For example, the medical apparatus may comprise a high-intensity focused ultrasound system for sonically treating or heating a subject.

In another embodiment the medical apparatus further comprises a radiation therapy system. This for example could be a gamma knife or a LINAC system.

In another aspect the invention provides for a method of operating a medical apparatus. The medical apparatus comprises an imaging chain component configured to be integrated into an imaging chain of the medical imaging system. The imaging chain is configured for acquiring measurement data during operation of the medical imaging system. The imaging chain component comprises a local computational system and local data communication interface for forming a local data connection configured for exchanging data between the local computational system and the medical imaging system. The method comprises causing the computational system to control the medical imaging system to initiate acquisition of the measurement data via the local data connection.

In another aspect, the invention provides for a computer program comprising local machine-executable instructions for execution by a local computational system that is integrated into an imaging chain component. The imaging chain component is configured to be integrated into an imaging chain of a medical imaging system. The imaging chain is configured for acquiring measurement data during operation of the medical imaging system. The imaging chain component comprises a local data communication interface for forming a local data connection that is configured for exchanging data between the local computational system and the medical imaging system. Execution of the local machine-executable instructions causes the computational system to control the medical imaging system to initiate acquisition of the measurement data via the local data connection.

It is understood that one or more of the aforementioned embodiments of the invention may be combined as long as the combined embodiments are not mutually exclusive.

As will be appreciated by one skilled in the art, aspects of the present invention may be embodied as an apparatus, method, or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.
A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Measurement data is defined herein as being recorded measurements made by a medical imaging system descriptive of a subject. The measurement data may be reconstructed into a medical image. A medical image is defined herein as being the reconstructed two-, three-, or four-dimensional visualization of anatomic data contained within the measurement data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical apparatus;
Fig. 2 illustrates a further example of a medical apparatus;
Fig. 3 shows a flow chart which illustrates a method of using the medical apparatus of Fig. 2;
Fig. 4 illustrates a further example of a medical apparatus;
Fig. 5 illustrates a further example of a medical apparatus; and
Fig. 6 illustrates a further example of a medical apparatus.

### DETAILED DESCRIPTION OF EMBODIMENTS

Like numbered elements in these figures are either equivalent elements or perform the same function. Elements which have been discussed previously will not necessarily be discussed in later figures if the function is equivalent.

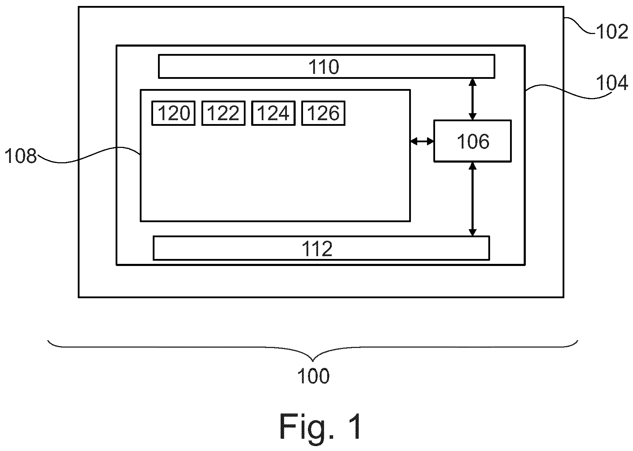

Fig. 1 illustrates an example of a medical apparatus 100. The medical apparatus 100 is shown as comprising an imaging chain component 102 that comprises a local control unit 104. The local control unit 104 comprises a local computational system 106. The local computational system 106 is in communication with a local memory 108.

The local computational system 106 is shown as comprising a local data communication interface 110 configured for forming a local data connection with a medical imaging system. The local computational system 106 is further shown as being connected to an optional network interface 112. The optional network interface 112 may for example be useful in forming network connections with other components such as a remote reconstructor system or a remote user interface or control system.

The local memory 108 is shown as containing local machine-executable instructions 120. The local machine-executable instructions 120 enable the computational system 106 to perform basic numerical and control tasks. The memory 108 is further shown as containing an initiated acquisition command 122 that the computational system 106 can send via the local data communication interface 110 to a medical imaging system. The memory 108 is further shown as optionally containing a system configuration data 124. The system configuration data 124 can be used to configure or modify the operation of a medical imaging system to acquire medical imaging data. The memory 108 is further shown as optionally containing system control commands 126. These system control commands 126 are an alternative to the system configuration data 124. The system control commands 126 may be used by the computational system 106 to directly control the medical imaging system. The system configuration data 124 and the system control commands 126 illustrate two optional alternatives. In one, the system configuration data 124 is used to configure a medical imaging system which is then started using the initiated acquisition commands 122. In the alternative, the initiated acquisition commands 122 are part of the system control commands 126 which are used to directly control the acquisition of the measurement data.

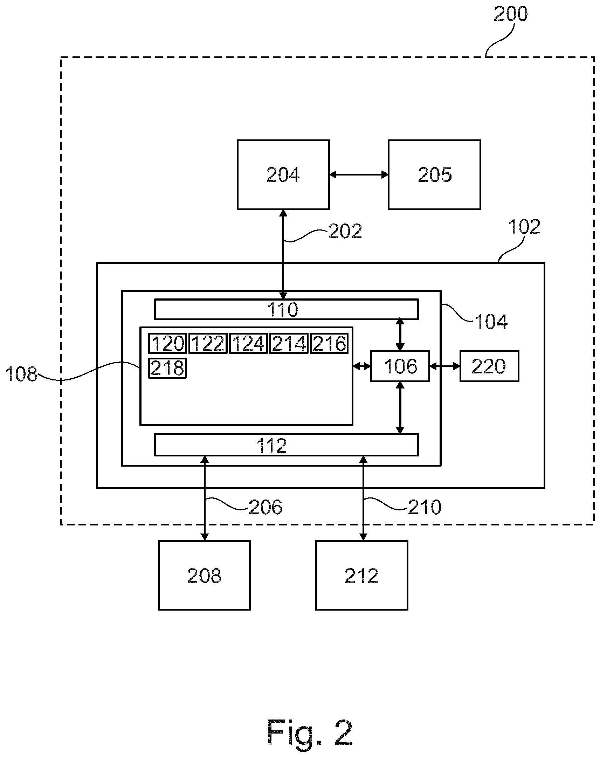

Fig. 2 illustrates a further example of a medical apparatus 200. The medical apparatus 200 is further shown as containing a system controller which is used to control a medical imaging system 205. The local data communication interface 110 is shown as having formed a local data connection 202 with the system controller 204.

The network interface 112 is shown as having formed an optional network connection 206 with a remote reconstructor system 208. Measurement data can be sent via the network connection 206 to be reconstructed into medical images. The network interface 112 is also shown as optionally forming a user interface connection with a remote user interface 212. The remote user interface 212 may for example be another computer, a tablet, a cell phone, a smartphone, or other computing device. In this case, the remote user interface 212 is used to send via the user interface connection 210 a set of modification data 214. The modification data 214 is used to modify the system configuration data 124 to provide modified configuration data 216.

The computational system 106 can then send the modified configuration data 216 to the system controller 204. After this, the initiated acquisition commands 122 can also be sent via the local data connection 202 to the system controller 204 to cause the medical imaging system 204 to acquire the measurement data 218. In this particular example, the imaging chain component 102 is shown as comprising a data measurement component 220. This may for example be a sensor or digitizer used to acquire the measurement data 218 directly. As the measurement data 218 is acquired directly by the imaging chain component 102 in this example, it is stored in the memory 108 and then transferred via the network connection 206 to the remote reconstructor system 208. If the optional remote reconstructor system 208 and optional user interface 212 are used, it can be seen that the data essentially bypasses the medical imaging system 205 and the control and configuration of the medical imaging system 205 has essentially been moved to the imaging chain component 102.

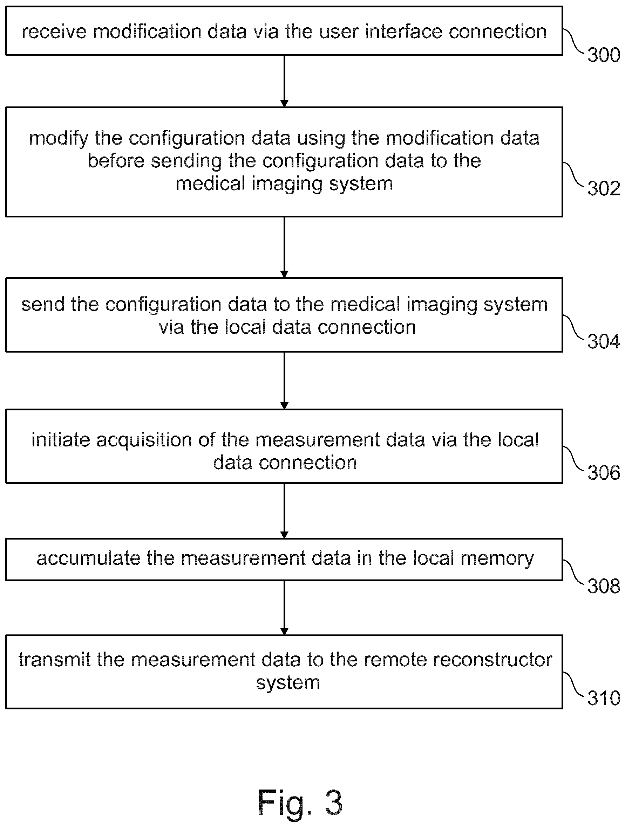

Fig. 3 shows a flowchart which illustrates a method of operating the medical apparatus 200 of Fig. 2. First, in step 300, the modification data 214 is received via the user interface connection 210. Next, in step 302, the system configuration data 124 is modified using the modification data 214 to provide the modified configuration data 216. Next, in step 304, the modified configuration data 216 is sent to the system controller 204 of the medical imaging system 205 via the local data connection 202. As an alternative, in step 304, the system configuration data 124 can be sent directly to the system controller 204 of the medical imaging system 205 without modification. Next, in step 306, acquisition of the measurement data 218 is started by sending the initiated acquisition commands 122 to the system controller 204 of the medical imaging system 205. Then, in step 308, the measurement data 218 is accumulated in the local memory 108. Finally, in step 310, the measurement data 218 is transmitted to the remote reconstructor system 208 via the network connection 206.

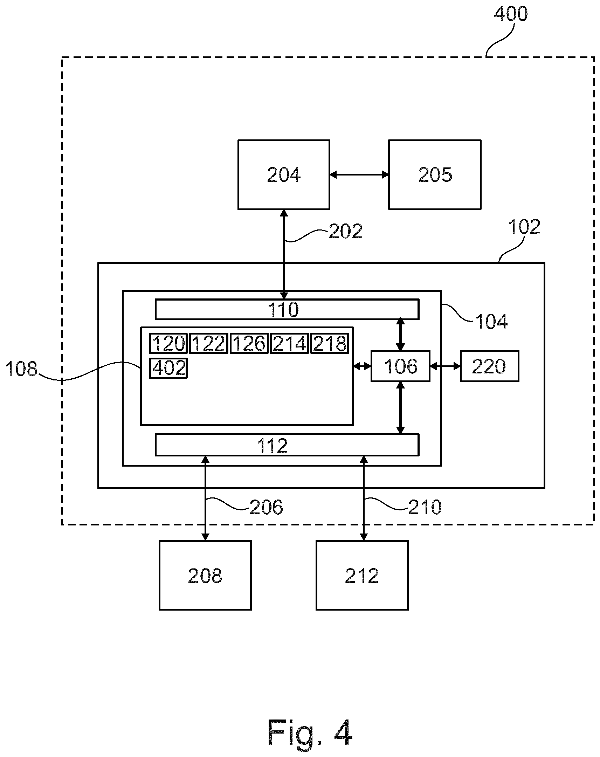

Fig. 4 illustrates a further example of a medical apparatus 400. The medical apparatus 400 is similar to the medical apparatus 200 depicted in Fig. 2 except that instead of configuring the medical imaging system 205 with the modified configuration data 216 the system control commands 126 are used to control the medical imaging system 205 directly. The memory 108 is again shown as containing the modification data 214. However, instead of modifying the configuration data the memory 108 is shown as containing modified system control commands 402. Once the initiated acquisition commands 122 are executed the modified system control commands 402 are used to control the medical imaging system 205 to acquire the measurement data 218.

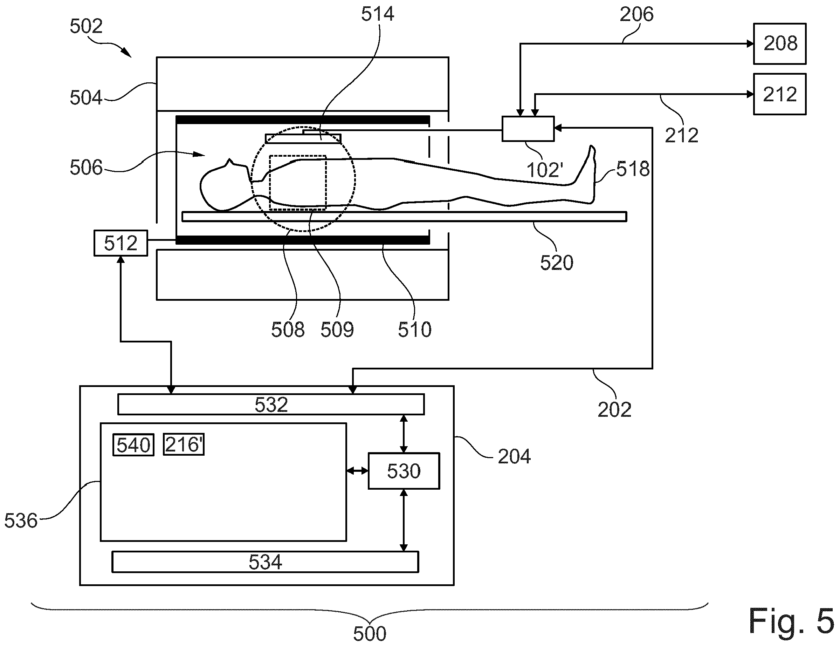

Fig. 5 illustrates a further medical apparatus 500 that is similar to the medical apparatus 200 depicted in Fig. 2. In this example the medical apparatus 500 additionally comprises a magnetic resonance imaging system 502 that is controlled by the system controller 204.

The system controller 204 is shown as being implemented using a processor 530. The processor 530 is shown as being connected to a network interface 532 that is used to form the local data connection with an imaging chain component 102'. In this example the imaging chain component is a transceiver. The processor is further in communication with a computer memory 536 and an optional user interface 534.

The magnetic resonance imaging system 502 comprises a magnet 504. The magnet 504 is a superconducting cylindrical type magnet with a bore 506 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 506 of the cylindrical magnet 304 there is an imaging zone 508 where the magnetic field is strong and uniform enough to perform magnetic resonance imaging. A field of view 509 is shown within the imaging zone 508. The k-space data that is acquired typically acquried for the field of view 309. The region of interest could be identical with the field of view 309 or it could be a sub volume of the field of view 509. A subject 518 is shown as being supported by a subject support 520 such that at least a portion of the subject 518 is within the imaging zone 508 and the field of view 509.

Within the bore 506 of the magnet there is also a set of magnetic field gradient coils 510 which is used for acquisition of k-space data within the imaging zone 508 of the magnet 504. The magnetic field gradient coils 510 connected to a magnetic field gradient coil power supply 512. The magnetic field gradient coils 510 are intended to be representative. Typically magnetic field gradient coils 510 contain three separate sets of coils for spatially encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging zone 508 is a radio-frequency coil 514 for manipulating the orientations of magnetic spins within the imaging zone 508 and for receiving radio transmissions from spins also within the imaging zone 508. The radio frequency antenna may contain multiple coil elements. The radio frequency antenna may also be referred to as a channel or antenna. The radio-frequency coil 514 is connected to a radio frequency transceiver 102'. The radio-frequency coil 514 and radio frequency transceiver 102' may be replaced by separate transmit and receive coils and a separate transmitter and receiver. It is understood that the radio-frequency coil 514 and the radio frequency transceiver 102' are representative. The radio-frequency coil 514 is intended to also represent a dedicated transmit antenna and a dedicated receive antenna. Likewise the transceiver 102' may also represent a separate transmitter and receivers. The radio-frequency coil 514 may also have multiple receive/transmit elements and the radio frequency transceiver 102' may have multiple receive/transmit channels.

As was mentioned above, in this example the transceiver 200' is the imaging chain component. In this example the transceiver 200' comprises an interface for the radio-frequency coil 514 that may include a digitizer and/or matching network. In other examples, the imaging chain component could also be the radio-frequency coil 514 or a coil interface. The transceiver 200' is connected to the hardware interface 532 via the local data connection 202. In this example the modified configuration data is modified pulse sequence commands 216'. When the transceiver 200' sends the initiated acquisition commands 122 via the local data connection 202 the processor 530 then executes the modified pulse sequence commands 216'. This then causes the magnetic resonance imaging system 502 to acquire the measurement data 218. In this case, the measurement data 218 is k-space data. In this arrangement the transceiver 200' sends the k-space data directly to the remote reconstructor system 208 via the network connection 206. The processor 530 still controls the magnetic resonance imaging system 502 during the acquisition of the k-space data, however, the configuration of the system and the processing of the data have been moved away from the system controller 204. This for example enables the upgrading of the magnetic resonance imaging system 502 by simply replacing the transceiver 200'. As was mentioned before, other components of the imaging chain such as the radio-frequency coil 514 or its interface may also be replaced.

In Fig. 5, a magnetic resonance imaging system 502 was used to illustrate an example. The magnetic resonance imaging system could be replaced with or combined with other medical imaging systems such as, a positron emission tomography system, a computed tomography system, a single photon emission tomography system, a digital x-ray system, or a a digital fluoroscopy system. The illustration in Fig. 5 also applies to medical apparatuses that include multiple types of medical imaging systems. The illustration also applied to various types of image guided therapy systems such as image guided radiation therapy or image guided high intensity focused ultrasound. In these cases, the imaging chain component could also configure and trigger the additional medical imaging system or therapy device.

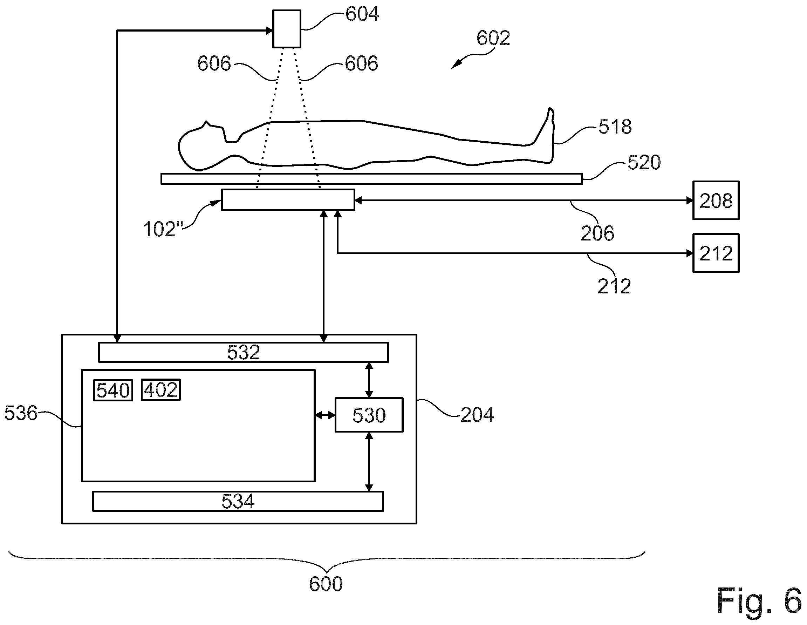

Fig. 6 illustrates a further example of a medical apparatus 600. The medical apparatus 600 comprises an X-ray machine 602. This comprises an X-ray source 604 configured for generating X-rays 606 that pass through the subject 518 into a flat-panel X-ray detector 102". The X-ray source 604 may comprise such components as an X-ray tube and a power supply for the X-ray tube. The flat-panel X-ray detector 102" is the imaging chain component. It can be seen as being connected to the remote reconstructor system 208 and the user interface 212. In this example, the initiated acquisition commands 122 are used to control the X-ray source 604 directly. When these are transmitted and received by the processor 530 it causes the X-ray source to be controlled by the modified system control commands 402.

In Fig. 6, an X-ray machine 602 was used to illustrate an example. The X-ray machine could be replaced or combined with other medical imaging systems such as, a positron emission tomography system, a computed tomography system, a single photon emission tomography system, a magnetic resonance imaging system, or a digital fluoroscopy system. The illustration in Fig. 6 also applies to medical apparatuses that include multiple types of medical imaging systems. The illustration also applied to various types of image guided therapy systems such as image guided radiation therapy or image guided high intensity focused ultrasound. The imaging chain component could also control the additional medical imaging system and/or therapy device.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical apparatus
- 102: imaging chain component
- 102': transceiver
- 102": flat panel x-ray detector
- 104: local control unit
- 106: local computational system
- 108: local memory
- 110: local data communication interface
- 112: network interface
- 120: local machine executable instructions
- 122: initiate acquisition commands
- 124: system configuration data
- 126: system control commands
- 200: medical apparatus
- 202: local data connection
- 204: system controller
- 205: medical imaging system
- 206: network connection
- 208: remote reconstructor system
- 210: user interface connection
- 212: remote user interface
- 214: modification data
- 216: modified configuration data
- 216': modified pulse sequence commands
- 218: measurement data
- 220: data measurement component
- 300: receive modification data via the user interface connection
- 302: modify the configuration data before sending the configuration data to the medical imaging system
- 304: sending the configuration data to the medical imaging system via the local data connection
- 306: initiate acquisition of the measurement data via the local data connection
- 308: accumulate the measurement data in the local memory
- 310: transmit the measurement data to the remote reconstructor system
- 400: medical apparatus
- 402: modified system control commands
- 500: medical apparatus
- 502: magnetic resonance imaging system
- 504: magnet
- 506: bore of magnet
- 508: imaging zone
- 509: field of view
- 510: magnetic field gradient coils
- 512: magnetic field gradient coil power supply
- 514: radio-frequency coil
- 518: subject
- 520: subject support
- 204: system controller
- 530: processor
- 532: network interface
- 534: user interface
- 536: computer memory
- 540: system machine executable instructions
- 600: medical apparatus
- 602: x-ray machine
- 604: x-ray source
- 606: x-rays

## Claims

1. A medical apparatus (100, 200, 400, 500, 600) comprising an imaging chain component (102, 102', 102") configured to be integrated into an imaging chain of a medical imaging system (205, 502, 602), wherein the imaging chain is configured for acquiring measurement data (218) during operation of the medical imaging system, wherein the imaging chain component comprises:
- a local memory (108) storing local machine executable instructions (120),
- a local computational system (106), and
- a local data communication interface (110) configured for forming a local data connection (202) for exchanging data between the local computational system and the medical imaging system, wherein execution of the local machine executable instructions causes the computational system to control the medical imaging system to initiate acquisition (306) of the measurement data via the local data connection.

2. The medical apparatus of claim 1, wherein the imaging chain component comprises a network interface (112) configured for forming a network connection (206) with a remote reconstructor system (208), wherein execution of the local machine executable instructions further causes the local computational system to:
- accumulate (308) the measurement data in the local memory; and
- transmit (310) the measurement data to the remote reconstructor system.

3. The medical apparatus of claim 1 or 2, wherein the local memory further comprises system configuration data (124) configured for configuring the medical imaging system to acquire the measurement data with the imaging chain, wherein execution of the machine executable instructions further causes the computational system to configure the medical imaging system by sending (304) the configuration data to the medical imaging system via the local data connection.

4. The medical apparatus of claim 3, wherein the imaging chain component is further configured for forming a user interface connection, wherein execution of the local machine executable instructions further causes the computational system to:
- receive (300) modification data (214) via the user interface connection; and
- modify (302) the configuration data using the modification data before sending the configuration data (216) to the medical imaging system.

5. The medical apparatus of any one of the preceding claims, wherein the local memory further comprises system control commands (126) configured for controlling the operation of the medical imaging system during the acquisition of measurement data, wherein execution of the machine executable instructions further causes the computational system to acquire the measurement data by controlling the medical imaging system via the local data connection with the system control commands.

6. The medical apparatus of any one of the preceding claims, wherein the imaging chain component comprises a data measurement component (220).

7. The medical apparatus of claim 6, wherein the medical imaging system comprises a magnetic resonance imaging system, and wherein the measurement data comprises k-space data.

8. The medical apparatus of claim 7, wherein the data measurement component is any one of the following: a magnetic resonance imaging coil, a wireless magnetic resonance imaging coil, and a magnetic resonance imaging coil interface (102') configured for receiving a magnetic resonance imaging coil

9. The medical apparatus of any one of claim 6, wherein the data measurement component is an X-ray detector (102").

10. The medical apparatus of claim 9, wherein the X-ray detector is any one of the following: a computed tomography detector, a digital X-ray detector, a flat panel x-ray detector (102"), and a flat panel wireless x-ray detector.

11. The medical apparatus of any one of claims 1 through 5, wherein the imaging chain component is any one of the following: an X-ray source, an X-ray tube, an X-ray tube power supply, a high voltage generator, and a controllable collimator.

12. The medical apparatus of claim 9, 10, or 11, wherein the medical imaging system further comprises an X-ray based diagnostic imaging system.

13. The medical apparatus of any one of the preceding claims, wherein the medical apparatus comprises the medical imaging system.

14. A method of operating a medical apparatus (100, 200, 400, 500, 600), wherein the medical apparatus comprises an imaging chain component (102, 102', 102") configured to be integrated into an imaging chain of a medical imaging system (205, 502, 602), wherein the imaging chain is configured for acquiring measurement data (218) during operation of the medical imaging system, wherein the imaging chain component comprises:
- a local computational system (106), and
- a local data interface (110) configured for forming a local data connection (202) for exchanging data between the local computational system and the medical imaging system, wherein the method comprises causing the computational system to control the medical imaging system to initiate acquisition (306) of the measurement data via the local data connection.

15. A computer program comprising local machine executable instructions (120) for execution by a local computational system (106) integrated into an imaging chain component (102, 102', 102"), wherein the imaging chain component is configured to be integrated into an imaging chain of a medical imaging system (205, 502, 602), wherein the imaging chain is configured for acquiring measurement data (218) during operation of the medical imaging system, wherein the imaging chain component comprises a local data interface (110) configured for forming a local data connection (202) for exchanging data between the local computational system and the medical imaging system, wherein execution of the local machine executable instructions causes the computational system to control the medical imaging system to initiate acquisition (306) of the measurement data via the local data connection.
